(19) Europäisches Patentamt
European Patent Office
Office européen des brevets

(11) EP 1 064 032 B1

(12) EUROPÄISCHE PATENTSCHRIFT

(45) Veröffentlichungstag und Bekanntmachung des Hinweises auf die Patenterteilung:
**25.02.2004 Patentblatt 2004/09**

(21) Anmeldenummer: **99919099.4**

(22) Anmeldetag: **15.03.1999**

(51) Int Cl.[7]: **A61L 27/00**, A61K 38/27

(86) Internationale Anmeldenummer:
**PCT/DE1999/000781**

(87) Internationale Veröffentlichungsnummer:
**WO 1999/047097 (23.09.1999 Gazette 1999/38)**

(54) **STOFFZUSAMMENSETZUNG ZUR GEWEBEBILDUNG**
MATERIAL COMPOSITION FOR THE FORMATION OF TISSUE
COMPOSITION DE MATIERE POUR LA FORMATION DE TISSU

(84) Benannte Vertragsstaaten:
**AT CH FR GB LI SE**

(30) Priorität: **19.03.1998 DE 19812195**

(43) Veröffentlichungstag der Anmeldung:
**03.01.2001 Patentblatt 2001/01**

(73) Patentinhaber: **STORCH, Uwe**
**D-45889 Gelsenkirchen (DE)**

(72) Erfinder: **STORCH, Uwe**
**D-45889 Gelsenkirchen (DE)**

(74) Vertreter:
**Haft, von Puttkamer, Berngruber, Czybulka**
**Patentanwälte**
**Franziskanerstrasse 38**
**81669 München (DE)**

(56) Entgegenhaltungen:
**EP-A- 0 466 552**
**WO-A-96/10374**
**US-A- 4 898 734**
**US-A- 5 653 996**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung bezieht sich auf ein Verfahren zur Herstellung eines Gewebe bildenden Implantats aus einem biodegradier baren Polyurethan gemäß Anspruch 1.

Eine Stoffzusammensetzung, die zu einem biodegradierbaren Polyurethan auspolymerisiert, ist bekannt (EP 0 531 487 B1). Mit ihr wird ein Implantat hergestellt, das insbesondere zur Auffüllung parodontaler Knochentaschen, Augmentation am Kieferknochen, als endodontische Füllung und zur Beseitigung von Knochendefekten verwendet wird. Dazu enthält es Hydroxylapatit als Füllstoff.

Die Wirkungsweise des Implantats beruht darauf, daß das Polyurethan-Implantat mit der Zeit hydrolysiert und resorbiert wird, so daß das Knochengewebe entsprechend nachwachsen kann. Demgemäß sollte die Resorptionsgeschwindigkeit des Implantats möglichst der Proliferationsgeschwindigkeit des Knochengewebes entsprechen. Tatsächlich weist Polyurethan jedoch eine erheblich langsamere Resorptionsgeschwindigkeit auf. Es sind zwar die verschiedensten Anstrengungen unternommen worden, die Resorptionsgeschwindigkeit des Polyurethan-Implantats zu erhöhen, von einer der Gewebeproliferation entsprechenden Resorptionsgeschwindigkeit ist man jedoch noch weit entfernt.

Aufgabe der Erfindung ist es, ein Verfahren zur Herstellung eines medizinischen Implantats bereitzustellen, das die Gewebebildung nicht wesentlich behindert.

Dies wird erfindungsgemäß mit dem im Anspruch 1 gekennzeichneten Verfahren erreicht.

Mit dem erfindungsgemäßen Verfahren wird ein Implantat gebildet, das aus einem offenzelligen Schaum besteht, also einem Polymerisat mit interkonnektierten, d. h. miteinander kommunizierenden Poren und dergleichen Hohlräumen. Diese Hohlräume werden vom Gewebe des Implantations-ortes entsprechend der Turnover- oder Proliferationsrate des Gewebes durchwachsen.

Der durch die Erfindung erzielte Vorteil liegt also einerseits in der plastischen Implantation der polymerisierbare Stoffzusammensetzung und in der dadurch erleichterten Anpassung an den Implantationsort, und andererseits darin, daß der hochgradig interkonnektierende Schaum sofort vom Gewebe durchwachsen werden kann.

Der offenporige Schaum besteht vorzugsweise aus einem biologisch degradierbaren Kunststoff. Er wird damit insbesondere von den Hohlräumen her abgebaut. Durch die große innere Oberfläche, die durch die Hohlräume gebildet wird, erfolgt eine so rasche Resorption des Kunststoffs, dass die Gewebebildung praktisch nicht behindert wird.

Als biologisch abbaubarer, offenzelliger Schaum wird vorzugsweise Polyurethan-Schaum verwendet.

Die Hydrolysebeständigkeit und Abbaurate des Polyurethan-Schaums hängt von der verwendeten Polyol-Komponente ab. Versuche hierzu haben folgende Rangfolge in der Hydrolysestabilität ergeben:
Polyether > Polycarprolactone > Polyester

Der hydrolytische Zerfall der Estergruppen wird durch saures Milieu begünstigt und verläuft wie folgt:

$$-\overset{\overset{O}{\|}}{C}-O- \quad + H_2O \rightarrow -OH + HO-\overset{\overset{O}{\|}}{C}-$$

Dadurch, daß beim Hydrolysevorgang endständige Carbonsäuregruppen entstehen, ist der Vorgang autokatalytisch. Bei Polyetherurethanen wird vorwiegend die Urethangruppe selbst hydrolysiert. Dabei entstehen zwei kürzere Ketten. Die eine besitzt eine endständige Hydroxylgruppe, die andere endet aminofunktionell.

$$\sim(O\text{-}CH_2)_4)_n)\text{-}O\text{-}CO\text{-}NH\text{-}R\text{-}NH\text{-}CO\text{-}O\sim + H_2O \rightarrow \sim(O\text{-}(CH_2)_4)_n\text{-}OH + H_2N\text{-}R\text{--}NH\text{-}CO\text{-}O\sim + CO_2$$

Durch den Zerfall von beigefügtem Polyactid-Oligomer werden in Abhängigkeit von der Kettenlänge desselben und seines Massenanteiles diese Prozesse beschleunigt. Gleiches gilt allgemein für die Freisetzung von Säuren, z.B. Phosphorsäure aus Hydroxylapatit. Durch oben beschriebenen Mechanismen sowie Vernetzungsgrad, Kristallinität, Porengröße und Polymerisationsgrad ist die Degradationsgeschwindigkeit des Werkstoffes einstellbar.

Da aromatische Gruppen eine karzinogene Wirkung besitzen können, werden erfindungsgemäß vorzugsweise zur Herstellung des Polyurethan-Schaums nur solche Polyol- oder Polyamin-Komponenten sowie nur solche Polyisocyanat-Komponenten verwendet, die keine aromatischen Gruppen aufweisen.

Als Polyol-Komponenten werden vorzugsweise langkettige aliphatische Verbindungen mit zwei oder drei Hydroxylgruppen mit einem Molekulargewicht zwischen 100 und 600 eingesetzt, insbesondere Diolester, ferner vorzugsweise Ricinusöl oder Castoröl. Dabei zeichnen sich Polyurethane, die unter Verwendung von Castoröl hergestellt werden, durch eine starke Gewebsadhäsion aus, also durch Ortsständigkeit des Implantationsmaterials, was in Abhängigkeit des Implantationsortes unter chirurgischen Aspekten wünschenswert ist.

Die Isocyanatgruppen des Polyisocyanats sind vorzugsweise durch wenigstens drei Methylengruppen voneinander getrennt. So hat sich Trimethylendiisocyanat als geeignet erwiesen. Ferner Diisocyanatcarbonsäuren, beispielsweise 2,6-Diisocyanathexansäure, die aus Lysin herstellbar ist.

Das Polyurethan wird aus einem Präpolymer aus der Polyol- bzw. Polyamin-Komponente einerseits und der Polyisocyanat-Komponente andererseits erhalten. Sowohl die Präpolymerisation wie die anschließende Polymerisation des Präpolymeren zum Polyurethan erfolgen in Masse, also ohne Verwendung von (toxischen). Lösungsmitteln.

Die Polymerisation des Präpolymer kann chemisch oder durch Bestrahlung initiiert werden. Zur chemischen Initiierung kann beispielsweise Wasserstoffperoxid verwendet werden.

Nach Initiierung der Polymerisation des Präpolymeren wird das Polyurethan bildende Gemisch plastisch implantiert. Um die Offenporigkeit des plastisch implantierten Polyurethans zu erhalten, kann die Polyisocyanat-Komponente in einem solchen Überschuß eingesetzt werden, daß während der Polymerisation des Polyurethans Kohlendioxid freigesetzt wird, das die Poren des Polyurethan-Schaums bildet. Das Molverhältnis der Isocyanatgruppen der Polyisocyanat-Komponente zu den Hydroxylgruppen der Polyol-Komponente (oder gegebenenfalls zu den Aminogruppen der Polyamin-Komponente) beträgt deshalb vorzugsweise mehr als zwei. Statt eines Polyisocyanat-Überschusses kann zur Porenbildung auch ein Schäummittel verwendet werden, das während der Polymerisation des Polyurethans Kohlendioxid oder ein anderes nicht toxisches Gas bildet.

Um einen steiferen Polyurethan-Schaum zu erhalten, hat es sich als vorteilhaft erwiesen, dem Präpolymer vor dessen Polymerisation ein aliphatisches Diisocyanat mit wenigstens drei Methylengruppen zwischen den beiden Isocyanatgruppen zuzusetzen, insbesondere die erwähnte 2,6-Diisocyanathexansäure. Ein steifer Schaum ist beispielsweise bei einem Knochenaufbau, etwa einer Augmentation im Kieferknochen, erwünscht.

Zur Porenbildung werden der polymerisierbaren Stoffzusammensetzung ferner wasserlösliche Feststoffpartikel zugesetzt, insbesondere Salze, wie Alkali- oder Erdalkali-Chloride oder Sulfate, wie Glucose. Die Feststoffpartikel werden aus dem implantierten Polymerisat herausgelöst. Die wasserlöslichen Feststoffpartikel dienen insbesondere zur Verbindung der Blasen innerhalb des Polymerisat, die durch das Gas gebildet werden, das während der Polymerisation entsteht. Die Menge der wasserlöslichen Feststoffpartikel kann beispielsweise 10 bis 60 vol.-% des Implantats betragen.

Das Porenvolumen des Schaumes sollte einerseits groß genug sein, um die Gewebeproliferation nicht zu beeinträchtigen, andererseits muß der Schaum eine hinreichende Festigkeit besitzen. Demgemäß sollte das Porenvolumen des Schaums mindestens 30 Vol.-%, insbesondere mindestens 50 Vol.-% betragen. Die durchschnittliche Porengröße kann 200 bis 600 μm betragen, insbesondere 350 bis 450 μm.

Die Porengröße kann unterschiedlich sein. Falls das Gewebe ein Knochengewebe ist, kann die Porengröße ähnlich der Eröffnungszone des hyalienen Knorpels bei der enchontralen Ossifikation beispielsweise 200 bis 600 μm betragen, während die Zwischenverbindungen der Poren entsprechend dem Querschnitt der Osteoklasten und Osteoblasten einen Durchmesser von weniger als 400 μm besitzen sollten.

Falls ein Gewebe mit langsamer Proliferationsgeschwindigkeit, z.B. Knochengewebe gebildet werden soll, das an ein Gewebe mit hoher Proliferationsgeschwindigkeit, beispielsweise Bindegewebe angrenzt, muß verhindert werden, daß das Bindegewebe in den Schaum hineinwachsen kann. Dies wird vorzugsweise dadurch erreicht, daß der offenporige Schaum auf der von dem zu regenerierenden Gewebe abgewandten Seite eine geschlossene Haut bildet. Eine solche Haut bildet sich häufig von selbst an der freien Oberfläche der implantierten Stoffzusammensetzung während der Polymerisation. Sie kann jedoch auch beispielsweise durch mechanisches Glätten der freien Oberfläche während der Polymerisation erzeugt werden.

Wenn mit dem erfindungsgemäßen Verfahren ein Knochengewebe gebildet werden soll, kann die polymerisierbare Stoffzusammensetzung ein Knochenersatz-Material als Füllstoff erhalten, insbesondere Hydroxylapatit, Tricalciumphosphat, Aluminiumoxid-Keramiken, aber auch sogenanntes Bioglas.

Ein wichtiger Aspekt des erfindungsgemäß hergestellten Implantats besteht darin, daß es als Wirkstoff-Carrier verwendet werden kann. Dazu sind die Wirkstoffe in die vorstehend beschriebenen wasserlöslichen Feststoffpartikel eingelagert und werden durch deren Auflösung freigesetzt.

Der Wirkstoff kann ein klassisches Arzneimittel, beispielsweise ein Antibiotikum, wie Tetracyclin, ein Corticoid oder dergleichen sein.

Um die Gewebebildung zu beschleunigen, wird als Wirkstoff jedoch vorzugsweise ein Gewebehormon verwendet, insbesondere von der Gruppe der Amelogine sowie BM(bone morphogenetic)-Proteine. Das Hauptproblem der klinischen Anwendung der Gewebshormone, das in deren Ortsständigkeit bzw. Plazierung beruht, wird durch das erfindungsgemäße Implantat gelöst.

Eine weitere Anwendungsmöglichkeit sind Implantate zur Tumorbehandlung. Dazu wird nach Entfernung des Tumorgewebes die erfindungsgemäß hergestellte Zusammensetzung implantiert, wobei sie als Carrier zur Abgabe des Tumornekrose-Faktors ausgebildet ist.

Wenn die wasserlöslichen Feststoffpartikel Neurotransmitter enthalten, kann das erfindungsgemäß hergestellte Implantat ferner auch für diesen Wirkstoff als Carrier verwendet werden. Damit kann Nervengewebe regeneriert

werden. So kann beispielsweise durch mikroinvasive Chirurgie eine Implantation an den betreffenden Stellen im Gehirn erfolgen z. B. um Epilepsie zu behandeln.

## Patentansprüche

1. Verfahren zur Herstellung eines Gewebe bildenden Implantats aus einem biodegradierbaren Polyurethan, wobei ein plastisches, lösungsmittelfreies Gemisch aus den Polyurethanpräpolymeren und festen Zusatzstoffen am Implantationsort appliziert und anschließend zum Implantat auspolymerisiert wird, **dadurch gekennzeichnet, dass** das Gemisch zur Bildung eines Implantats aus einem offenporigen Polyurethanschaumstoff als festen Zusatzstoff wasserlösliche Feststoffpartikel enthält.

2. Verfahren nach Anspruch 1, **dadurch gekennzeichnet, dass** der Gehalt der wasserlöslichen Feststoffpartikel in dem Gemisch 10 bis 60 Vol.-% beträgt.

3. Verfahren nach Anspruch 1 oder 2, **dadurch gekennzeichnet, dass** wasserlösliche Feststoffpartikel verwendet werden, die einen Wirkstoff enthalten.

4. Verfahren nach einem der vorstehenden Ansprüche, **dadurch gekennzeichnet, dass** eine geschlossene Haut auf der von dem sich bildenden Gewebe abgewandten Seite des am Implantationsort applizierten Gemisches gebildet wird.

5. Verfahren nach einem der vorstehenden Ansprüche, **dadurch gekennzeichnet, dass** das Molverhältnis der Isocyanatgruppen der Polyisocyanat-Komponente zu den Hydroxylgruppen der Polyolkomponente oder zu den Aminogruppen der Polyamin-Komponente des Polyurethans mehr als 2 beträgt.

6. Verfahren nach einem der vorstehenden Ansprüche, **dadurch gekennzeichnet, dass** die Polyol-Komponente des Polyurethans eine aliphatische Verbindung mit zwei oder drei Hydroxylgruppen und einem Molekulargewicht zwischen 100 und 600 ist.

7. Verfahren nach einem der vorstehenden Ansprüche, **dadurch gekennzeichnet, dass** die Polyisocyanat-Komponente des Polyurethans eine aliphatische Verbindung mit wenigstens zwei durch mindestens drei Methylengruppen getrennten Isocyanatgruppen ist.

## Claims

1. Process for the manufacture of an implant forming tissue, made of a biodegradable polyurethane, whereby a plastic, solvent-free mixture of the polyurethane prepolymers and solid additives are applied at the implantation location and then polymerised out to form the implant, **characterised in that** the mixture for the formation of an implant from an open-pore polyurethane foam material contains water-soluble solid particles as a solid additive.

2. Process according to Claim 1, **characterised in that** the content of the water-soluble solid particles in the mixture amounts to 10 to 60 % by volume.

3. Process according to Claim 1 or 2, **characterised in that** water-soluble solid particles are used which contain an active substance.

4. Process according to one of the foregoing claims, **characterised in that** a closed skin is formed on the side of the mixture applied at the implantation location which is turned away from the tissue which is forming.

5. Process according to one of the foregoing claims, **characterised in that** the molar ratio of the isocyanate groups of the polyisocyanate component to the hydroxyl groups of the polyol component or to the amino groups of the polyamine component of the polyurethane amounts to more than 2.

6. Process according to one of the foregoing claims, **characterised in that** the polyol component of the polyurethane is an aliphatic compound with two or three hydroxyl groups and a molecular weight between 100 and 600.

7. Process according to one of the foregoing claims, **characterised in that** the polyisocyanate component of the polyurethane is an aliphatic compound with at least two isocyanate groups separated by at least three methylene groups.

**Revendications**

1. Procédé de fabrication d'un implant constitué d'un polyuréthane biodégradable et générant du tissu, un mélange plastique sans solvant constitué de prépolymères de polyuréthane et d'additifs stables étant appliqué sur l'emplacement d'implantation puis polymérisé en implant,
**caractérisé en ce que** le mélange destiné à la formation d'un implant en une mousse de polyuréthane à pores ouverts sous forme d'additif stable contient des particules solides hydrosolubles.

2. Procédé selon la revendication 1, **caractérisé en ce que** la teneur en particules solides hydrosolubles du mélange est comprise entre 10 et 60 % en volume.

3. Procédé selon la revendication 1 ou 2, **caractérisé en ce que** des particules solides hydrosolubles contenant un principe actif sont utilisées.

4. Procédé selon l'une quelconque des revendications précédentes,
**caractérisé en ce qu'**une peau fermée est formée sur le côté du mélange appliqué sur l'emplacement d'implantation qui est tourné dans le sens contraire du tissu généré.

5. Procédé selon l'une quelconque des revendications précédentes,
**caractérisé en ce que** le rapport molaire des groupes isocyanate du composant polyisocyanate par rapport aux groupes hydroxyde du composant polyol ou par rapport aux groupes amino du composant polyaminé du polyuréthane est supérieur à 2.

6. Procédé selon l'une quelconque des revendications précédentes,
**caractérisé en ce que** le composant polyol du polyuréthane est un composé aliphatique avec deux ou trois groupes hydroxyle et une masse moléculaire comprise entre 100 et 600.

7. Procédé selon l'une quelconque des revendications précédentes,
**caractérisé en ce que** le composant polyisocyanate du polyuréthane est un composé aliphatique avec au moins deux groupes isocyanate séparés par au moins trois groupes méthylène.